# EUROPEAN PATENT SPECIFICATION

(11) **EP 3 286 331 B1**
(45) Date of publication and mention of the grant of the patent: **14.12.2022**
(21) Application number: 16722806.3
(22) Date of filing: 25.04.2016
(51) Int. Cl.: C12Q 1/6832

(54) **METHOD FOR HYBRIDIZING A NUCLEIC ACID MOLECULE**
VERFAHREN ZUR HYBRIDISIERUNG EINES NUKLEINSÄUREMOLEKÜLS
PROCÉDÉ D'HYBRIDATION D'UNE MOLÉCULE D'ACIDE NUCLÉIQUE

(30) Priority: 24.04.2015 EP 15164962
(43) Date of publication of application: 28.02.2018
(73) Proprietor: Qiagen GmbH, 40724 Hilden (DE)
(72) Inventor: KORFHAGE, Christian, 40724 Hilden (DE); FRICKE, Evelyn, 40724 Hilden (DE)
(74) Representative: Witte, Weller & Partner Patentanwälte mbB
(86) International application number: PCT/EP2016/059167
(87) International publication number: WO 2016/170181

(56) References cited:
- US-A1- 2004 132 061
- US-A1- 2007 190 548
- US-A1- 2010 075 328
- US-A1- 2015 045 254
- YI JIZU ET AL: "Molecular Zipper: a fluorescent probe for real-time isothermal DNA amplification", NUCLEIC ACIDS RESEARCH, INFORMATION RETRIEVAL LTD, vol. 34, no. 11, 1 January 2006 (2006-01-01), pages E81-E85, XP002434420, ISSN: 0305-1048, DOI: 10.1093/NAR/GKL261
- LANE S ET AL: "Amplicon secondary structure prevents target hybridization to oligonucleotide microarrays", BIOSENSORS AND BIOELECTRONICS, ELSEVIER BV, NL, vol. 20, no. 4, 1 November 2004 (2004-11-01), pages 728-735, XP004633820, ISSN: 0956-5663, DOI: 10.1016/J.BIOS.2004.04.014
- Malhotra S Sharma S ET AL: "Molecular Methods in Microbiology and their Clinical Application", Journal of Molecular and Genetic Medicine, vol. 08, no. 04, 1 January 2014 (2014-01-01), XP055608714, DOI: 10.4172/1747-0862.1000142

## Description

The present invention is directed to a method for hybridizing a nucleic acid molecule to another nucleic acid molecule and to a use of a nucleic acid molecule to reduce and/or avoid the formation of hairpin structures of a nucleic acid target molecule.

### FIELD OF THE INVENTION

The present invention relates to the field of molecular biology, more particularly to the hybridization of nucleic acid molecules.

### BACKGROUND OF THE INVENTION

In the field of molecular or recombinant biology for many tasks it is necessary to hybridize nucleic acid molecules against each other.

Nucleotide hybridization (in the following "hybridization") is a bimolecular reaction where single-stranded deoxyribonucleic acid (DNA) or ribonucleic acid (RNA) molecules of complementary or at least partially complementary nucleotide sequences anneal to each other. DNA replication and transcription of DNA into RNA both rely upon hybridization, as do molecular biology techniques including Southern blots and Northern blots, the polymerase chain reaction (PCR), and most approaches to DNA sequencing. In other techniques, short DNA sequences are hybridized to cellular mRNAs to identify expressed genes. Antisense RNA is also used to bind to undesired mRNA, preventing the ribosome from translating the mRNA into protein.

Methods for hybridizing a nucleic acid molecule are known from US 2015/045254, US 2007/190548, US 2004/132067, and Yi et al. (2006 Nucleic Acid Research Vol. 34, No. 11 p. 1-5, and Malhotra et al. (2014) J. Mol. Genet. Med. Vol. 4 Is. 4 p. 1-9.

However, each of the single-stranded nucleic acid molecules involved in the hybridization reaction may form secondary structures such as stem-loops or hairpins, respectively, by intramolecular base pairing. It occurs when two regions of the same strand, usually complementary in nucleotide sequence when read in opposite directions, base-pair to form a double helix that ends in an unpaired loop. The resulting structure is a key building block of many RNA secondary structures.

The formation of a hairpin structure depends on the stability of the resulting helix and loop regions. The first prerequisite for the formation of a hairpin structure is the presence of a sequence that can fold back on itself resulting in a paired doubled helix. The stability of this helix is determined by its length, the number of mismatches or bulges it contains and the base composition of the paired region. Pairings between guanine and cytosine have three hydrogen bonds and are more stable compared to adenine-uracil pairings, which have only two. In RNA, adenine-uracil pairings featuring two hydrogen bonds are equal to an adenine-thymine bond of the DNA.

Hairpin structures of circular DNA are stabilized by so-called supercoiling. Supercoiling refers to the over- or under-winding of a nucleic acid strand and is an expression of the strain on that strand.

The formation of intramolecular secondary structures, such as hairpin structures, negatively influences the effectivity of hybridization processes between two individual nucleic acid molecules. The more and stable hairpin structures are formed in a nucleic acid molecule, the slower the formation of hybrids occurs between such a nucleic acid molecule and another nucleic acid molecule.

The formation of intramolecular secondary structures is a particular problem in hybridization reactions where one of the two nucleic acid molecules involved in the hybridization reaction is immobilized on a solid support. Gao et al. (2006), Secondary structure effects on DNA hybridization kinetics: a solution versus surface comparison, Nucleic Acids Res. 5,34(11):3370-3377, describe that in different systems a hybridization speed can be measured which is 20-fold to 40-fold reduced if one of the nucleic acid strands to be hybridized was immobilized on a solid support. If e.g. short oligonucleotides are immobilized on a solid support and a long single-stranded nucleic acid molecule that is non-immobilized in a surrounding solution, which should be hybridized to the immobilized oligonucleotide, the following situation can be observed: The hybridization region is short because of the shortness of the oligonucleotides which were immobilized to the solid support. A frequently used length of the hybridizing part of the immobilized oligonucleotide molecules is about 15 to 35 nucleotides. On the other side, long nucleic acid single strands are in solution. A frequently used length of such nucleic acid single strands is 200 to 500 nucleotides. Long nucleic acid single strands have a higher tendency to form hairpin structures than short nucleic acid single strands. The nucleic acid single strands which form hairpin structures slow the hybridization speed and reduce the number of hybridized double strands. If long nucleic acid molecules are immobilized on the surface of the solid support, for example in a BAC array, also the immobilized single strand will form hairpin structures resulting in similar conflicts of reducing the hybridization effectivity.

Against this background, it is an object underlying the present invention to provide a method for hybridizing a nucleic acid molecule to another nucleic acid molecule with a higher efficiency as this is the case with the current methods in the art.

The present invention satisfies these and other needs.

### SUMMARY OF THE INVENTION

The present invention provides a method for hybridizing a nucleic acid molecule, comprising
i) providing a reaction mixture comprising
   - at least one first nucleic acid molecule comprising at least partially hybridized against each other
      - a sense nucleic acid strand having
         - at least a first nucleotide sequence, and
         - at least a second nucleotide sequence;
      - an antisense nucleic acid strand having at least a third nucleotide sequence being at least partially complementary to said a least second nucleotide sequence and being at least partially non-complementary to said at least first nucleotide sequence;
   - at least one second nucleic acid molecule having a fourth nucleotide sequence being at least partially complementary to said first nucleotide sequence;
   - hybridization buffer;
ii) incubating said reaction mixture under conditions allowing said at least one first nucleic acid molecule to hybridize to said at least one second nucleic acid molecule via a hybridization reaction between said at least first nucleotide sequence and said at least fourth nucleotide sequence,
   characterized in that said at least one first nucleic acid molecule is provided as follows:
   - providing said sense nucleic acid strand,
   - hybridizing at least one primer oligonucleotide to at least a first segment of said second nucleotide sequence, said at least first segment is located upstream and/or downstream of said first nucleotide sequence,
   - extending said primer oligonucleotide by a nucleotide polymerase to generate said antisense nucleic acid strand, and
said least one second nucleic acid molecule is linked to a solid support.

The inventors have developed a method for hybridizing a nucleic acid molecule to another nucleic acid molecule where surprisingly the formation of secondary structures such as hairpin structures of the nucleic acid molecules to be hybridized is significantly reduced. This results in considerable high hybridization efficiency.

As used herein, "hybridizing" or "hybridization" refers to the phenomenon in which single-stranded nucleic acid molecules such as deoxyribonucleic acid (DNA) or ribonucleic acid (RNA) molecules of a particular nucleotide sequence anneal to DNA or RNA molecules comprising complementary or partially complementary nucleotide sequences, thereby forming a hybrid of the two single-stranded nucleic acid sequences resulting in a double-stranded nucleic acid molecule. The hybridization process is based on the formation of hydrogen bonds between complementary bases of the nucleotides. Nucleic acid molecules can be perfectly hybridized against each other in case of perfect or full complementarity, i.e. each and every nucleotide or base in the first molecule is opposed by the complementary nucleotide in the second molecule. Nucleic acid molecules can be partially hybridized against each other in case of imperfect or partial complementarity, i.e. not each and every nucleotide or base in the first molecule is opposed by the complementary nucleotide or base in the second molecule, however a sufficient number of nucleotides or bases are opposed by the respective complementary partners still allowing the formation of a nucleic acid hybrid. Besides the complementarity of the nucleotide sequences, the lengths of the nucleotide sequences, the mobility or immobility of the nucleotide sequences, a hybridization reaction requires additional specific conditions to be fulfilled determined by the pH value, ion strength, temperature, time, etc.

As used herein, a "nucleic acid" or "nucleic acid molecule" may be generally either DNA or RNA, single- or double-stranded, linear or circular, unless specified otherwise.

As used herein, the term "nucleotide" refers to the monomers or subunits of nucleic acids like DNA and RNA. Nucleotide may be synonymously used for nucleoside or nucleoside (mono-, di-, tri-) phosphate, and includes the deoxyribose derivatives, i.e. the dNTPs.

As used herein, a "nucleotide sequence" refers to a catenation of nucleotides by phosphodiester bridges.

As used herein, "partially complementary" means a nucleotide sequence which is sufficiently complementary to the referring sequence, thereby allowing a hybridization reaction between such sequences. Such partially complementary nucleotide sequences can be ≥ 50 %, preferably ≥ 60 %, more preferably ≥ 70 %, more preferably ≥ 80 %, more preferably ≥ 90 %, more preferably ≥ 95 %, more preferably ≥ 99 %, or 100 % complementary to each other.

At least "partially non-complementary" means a nucleotide sequence which is sufficiently non-complementary to the referring sequence, thereby not allowing a hybridization reaction between such sequences. such partially non-complementary nucleotide sequences can be ≥ 50 %, preferably ≥ 60 %, more preferably ≥ 70 %, more preferably ≥ 80 %, more preferably ≥ 90 %, more preferably ≥ 95 %, more preferably ≥ 99 %, or 100 % non-complementary to each other.

As used herein, said "first nucleic acid molecule" is a partially double-stranded and partially single-stranded nucleic acid molecule. This is because the third nucleotide sequence of the antisense strand is not complementary or at least not sufficiently complementary to hybridize to the first nucleotide sequence of the sense strand. Said first nucleic acid molecule is - at least partially - single-stranded in regions of the sense strand comprising or consisting of the first nucleotide sequence. Said first nucleic acid molecule is - at least partially - double-stranded in regions where said second nucleotide sequence of the sense nucleic acid strand and the third nucleotide sequence of the antisense strand are complementary and hybridized against each other. The single-stranded region determined by the first nucleotide sequence is provided for the hybridization of the first nucleic acid molecule to the second nucleic acid molecule due to the at least partial complementarity of the first and the fourth nucleotide sequence. The third nucleotide sequence is at least partially complementary to said at least second nucleotide sequence. However, at the same time the third nucleotide sequence is not complementary or at least not sufficiently complementary to hybridize to the first nucleotide sequence. It shall be understood that the sense and/or antisense strand of the first nucleic acid molecule may comprise additional nucleotide sequences besides the first and the second nucleotide sequences. The first nucleic acid molecule has preferably, but not exclusively, a circular configuration.

As used herein, "at least partially hybridized against each other" means that ≥ 10 %, or ≥ 20 %, or ≥ 30 %, or ≥ 40 %, or ≥ 50 %, or ≥ 60 %, or ≥ 70 %, or ≥ 80 %, or ≥ 90 %, or 100 % of a nucleotide sequence, in particular the second nucleotide sequence of the sense strand of the first nucleic acid molecule, is covered by another nucleotide sequence, in particular the third nucleotide sequence of the antisense strand of the first nucleic acid molecule.

As used herein, the "second nucleic acid molecule" may be single- or at least partially double-stranded, DNA or RNA, free in solution or immobilized by linkage to a surface of a solid support. The second nucleic acid molecule comprises a fourth nucleotide sequence that is at least partially complementary to said first nucleotide sequence of the sense strand of the first nucleic acid molecule. The second nucleic acid molecule may comprise other nucleotide sequences besides the fourth nucleotide sequence. The second nucleic acid molecule has preferably, but not exclusively, a linear configuration.

The sense strand of the first nucleic acid molecule, i.e. the sum of the first and second nucleotide sequence, and any of the strands of the second nucleic acid molecule, i.e. the sum of the fourth nucleotide sequence and any additional nucleotide sequence the second nucleic acid may comprise, can have length of ≥ 15, or ≥ 40, or ≥ 100, or ≥ 200, or ≥ 300, or ≥ 400, or ≥ 500, or ≥ 1000, or ≥ 2000, or ≥ 3000, or ≥ 4000, or ≥ 5000 nucleotides. Hereof, the first nucleotide sequence of the first nucleic acid molecule and/or the fourth nucleotide sequence of the second nucleic acid molecule can have lengths of preferably ≥ 5, preferably ≥ 10, more preferably ≥ 15, more preferably ≥ 20, more preferably ≥ 25, more preferably ≥ 30, or ≥ 35, or ≥ 40, or ≥ 50, or ≥ 100, or ≥ 200, or ≥ 300, or ≥ 400 nucleotides. The first and the fourth nucleotide sequences can hybridize with each other over their entire length or only over a length of ≥ 5, or ≥ 10, or ≥ 15, or ≥ 20, or ≥ 25, or ≥ 30, or ≥ 35, or ≥ 40, or ≥ 50, or ≥ 100, or ≥ 200, or ≥ 300, or ≥ 400 nucleotides.

The first, second, third, and fourth and any other nucleotide sequences of the invention may comprise mononucleotide sequences, such as polyA, polyT, polyC, polyG, polyU, polyl etc., or complex nucleotide sequences comprising two, three, four, or more different types of nucleotides.

The sense strand of the first nucleic acid molecule and/or any of the strands of the second nucleic acid molecule can have the function of probes or targets in any molecular arrangement to be established via the hybridization reaction of the method according to the invention. A suitable molecular arrangement is an array. Examples are two-color arrays, NGS, microarray, BAC arrays etc.

The first and/or second nucleic acid molecule can be provided in purified form, partially purified form or contained in a raw lysate.

As used herein, "hybridization buffer" refers to a solution providing conditions, in particular salts and any other compounds, in an appropriate composition and concentration, allowing the hybridization of complementary or at least partially complementary nucleotide sequences, such as the first with the fourth nucleotide sequences, and the second with the third nucleotide sequences.

"At least one", as used herein, refers to one entity at the minimum, e.g. one nucleic acid molecule, one nucleotide sequence etc. However, there can be more than one entity, e.g. two, three, four, five, six, seven, eight, nine, ten, one hundred, one thousand, ten thousand etc. entities.

Unless otherwise specified, the individual steps of the method according to the invention may be carried out sequentially or in another embodiment simultaneously/in parallel, respectively. In an embodiment of the method according to the invention, said at least one first nucleic acid molecule is a circular nucleic acid molecule.

The inventors have realized that the method according to the invention results in a higher efficiency of hybridizing a first nucleic acid molecule to a second nucleic acid molecule via the at least partial complementarity of their nucleotide sequences than this is the case in prior art hybridization methods. The antisense nucleic acid strand of the first nucleic acid molecule covers or masks the regions of the sense nucleic acid strand not involved or necessary for the hybridization to the second nucleic acid molecule. By this coverage or masking the regions of the sense nucleic acid strand not involved or necessary for the hybridization are not susceptible for the formation of strand internal hybridization reactions or secondary structures, such as hairpin structures, which might otherwise prevent or complicate the hybridization of the first nucleic acid molecule to the second nucleic acid molecule.

Because of the higher hybridization efficiency, a fewer amount of the first nucleic acid molecule needs to be provided in the reaction mixture than it would be required if the second nucleotide sequence would not be covered by the antisense nucleic acid strand.

In an embodiment of the method according to the invention said at least one first nucleic acid molecule is a circular nucleic acid molecule.

Circular nucleic acid molecules are particularly vulnerable to secondary structures such as hairpin structures as they are stabilized by a supercoiling process. Therefore, in the art hybridizing of circular nucleic acid molecules is particularly difficult. However, the method according to the invention comes to the rescue. With this embodiment, even a circular first nucleic acid molecule can be hybridized to the second nucleic acid molecule with high efficiency.

As used herein, "circular nucleic acid molecule" refers to a nucleic acid molecule without any free 3' and/or 5' termini or to a nucleic acid molecule where the 3' and 5' intramolecular termini were previously covalently linked to each other, thereby conferring the nucleic acid template a ring- or loop-like or similar structure.

In the method according to the invention, said at least one second nucleic acid molecule is linked to a solid support.

Especially for the immobilization of nucleic acid molecules to solid supports or the surfaces thereof by hybridization reactions, the formation of hairpin structures is a particular barrier. However, with this measure of the invention an efficient hybridization even to solid supports becomes possible.

As used herein, a "solid support" refers to any support comprising a surface which may be planar or curved and being capable of receiving and linking nucleic acid molecules. Solid supports of all kinds typically used in the field of immobilization of nucleic acid molecules are encompassed, such as planar chips, beads, capillaries etc. The support may be made of metal, glass, silica, plastics etc. It may also comprise a coated surface. The solid support may also comprise a soft and/or flexible surface.

In another embodiment of the method according to the invention before and/or during said extension, a blocking oligonucleotide is hybridized to at least a second segment of said second nucleotide sequence, said second segment is located downstream to said first segment, preferably adjacent to the latter, and preferably said polymerase exhibits no strand displacement activity and no 5'-3' exonuclease activity.

With this measure precautions are taken ensuring that the first nucleotide sequence of the sense strand remains single-stranded and, therefore, available for the hybridization to the fourth nucleotide sequence of the second nucleic acid molecule. This embodiment is particularly preferred if the first nucleic acid molecule is a circular nucleic acid molecule.

In this embodiment, the blocking oligonucleotide is located downstream of the first nucleotide sequence, preferably directly adjacent to the latter. The 3' terminus of the blocking oligonucleotide is facing the 3' terminus of the first nucleotide sequence. Because of the terminal blockage, the blocking oligonucleotide cannot be extended by the polymerase. Because of the use of a polymerase without strand displacement activity and without 5'-3' exonuclease activity, the primer extension reaction stops as soon as the polymerase has reached the 5' terminus of the 3' terminally blocked oligonucleotide.

In another embodiment of the method according to the invention, said fourth nucleotide sequence is longer than said first nucleotide sequence.

This measure has the advantage that the hybridization reaction between the fourth nucleotide sequence and the first nucleotide sequence is energetically favored over an intramolecular hybridization reaction within the first nucleotide sequence and the formation of hairpin structures. The effectivity of the hybridization reaction between the fourth nucleotide sequence and the first nucleotide sequence is herewith again increased. "Longer" in this context means that the fourth nucleotide sequence is composed of more nucleotides than the first nucleotide sequence.

In another embodiment of the method according to the invention, said at least one first nucleic acid molecule and said at least one second nucleic acid molecule are DNA, preferably said at least one second nucleic acid molecule is a single-stranded nucleic acid molecule.

This measure has the advantage that the method according to the invention is adapted to such kind of nucleic acid molecules which are of highest significance in hybridization reactions.

With this measure precautions are taken ensuring that the first nucleotide sequence of the sense strand remains single-stranded and, therefore, available for the hybridization to the fourth nucleotide sequence of the second nucleic acid molecule. This embodiment is particularly preferred if the first nucleic acid molecule is a circular nucleic acid molecule.

In this embodiment, the blocking oligonucleotide is located downstream of the first nucleotide sequence, preferably directly adjacent to the latter. The 3' terminus of the blocking oligonucleotide is facing the 3' terminus of the first nucleotide sequence. Because of the terminal blockage, the blocking oligonucleotide cannot be extended by the polymerase. Because of the use of a polymerase without strand displacement activity and without 5'-3' exonuclease activity, the primer extension reaction stops as soon as the polymerase has reached the 5' terminus of the 3' terminally blocked oligonucleotide.

In another embodiment of the method according to the invention, said at least first nucleic acid molecule is provided as follows:
- providing a double-stranded nucleic acid molecule comprising
   - said sense nucleic acid strand and said antisense nucleic acid strand at least partially hybridized against each other, and
   - an interposed antisense strand, said interposed antisense strand is at least partially hybridized to said first nucleotide sequence, and
- degradation of said interposed antisense strand by a nuclease.

With this measure and alternative approach for generating the single-stranded section of the first nucleic acid molecule is realized.

The interposed antisense strand can be covalently linked to the antisense strand by means of ordinary phosphodiester bonds so that the antisense and the interposed antisense strands are seamlessly connected to each other. The nucleotide sequence of the interposed antisense strand may then be complementary or at least partially complementary to the first nucleotide sequence. The interposed antisense strand is then removed from the first nucleic acid molecule by the nuclease activity, such as an exonuclease, endonuclease and/or glycosylase activity, e.g. exerted by an N-uracilglycosylase.

In another embodiment of the method according to the invention, said fourth nucleotide sequence is longer than said first nucleotide sequence.

This measure has the advantage that the hybridization reaction between the fourth nucleotide sequence and the first nucleotide sequence is energetically favored over an intramolecular hybridization reaction within the first nucleotide sequence and the formation of hairpin structures. The effectivity of the hybridization reaction between the fourth nucleotide sequence and the first nucleotide sequence is herewith again increased. "Longer" in this context means that the fourth nucleotide sequence is composed of more nucleotides than the first nucleotide sequence.

In another embodiment of the method according to the invention, said at least one first nucleic acid molecule and said at least one second nucleic acid molecule are DNA, preferably said at least one second nucleic acid molecule is a single-stranded nucleic acid molecule.

This measure has the advantage that the method according to the invention is adapted to such kind of nucleic acid molecules which are of highest significance in hybridization reactions.

In another embodiment the method according to the invention comprises the following further step:
iii) removing said antisense strand, preferably by denaturation, further preferably by using a polymerase with strand displacement activity.

After the hybridization of the first nucleic acid molecule to the second nucleic acid molecule, the formation of intramolecular secondary structures within the second nucleotide sequence is energetically no longer favored. Therefore, the antisense strand is no longer necessary and became inoperable.

This measure has therefore the advantage that the second nucleotide sequence of the sense strand becomes accessible and, therefore, available for any specific functions such as amplification reactions, subsequent hybridization reactions with other nucleic acid molecules such as probes or primers, or for other reactions, e.g. with capture molecules, antibodies, dyes, markers etc.

The antisense strand can be removed by denaturation or via a strand displacement by using a polymerase with strand displacement activity. A polymerase with strand displacement activity is preferably used if the first nucleic acid molecule is a circular nucleic acid molecule. After the polymerase has synthesized the antisense strand and - because of the circular configuration of the first nucleic acid molecule - abuts the 5' terminus of the primer oligonucleotide the stranded displacement activity peals-off and thereby removes the antisense strand.

In another embodiment of the method according to the invention, said solid support comprises a material selected from the group consisting of: metal, glass, silica, plastics, and/or preferably is selected from the group consisting of: chips, beads, capillaries.

This measure has the advantage that the method according to the invention is adapted to any kind of material which is commonly used in molecular immobilization tasks.

Another subject matter of the present invention is the use of a nucleic acid antisense strand having a third nucleotide sequence being at least partially complementary to a second nucleotide sequence and being at least partially non-complementary to a first nucleotide sequence, to reduce and/or avoid the formation of hairpin structures of a nucleic acid target molecule comprising said first nucleotide sequence and said second nucleotide sequence, said nucleic acid target molecule intended to be hybridized to a nucleic acid probe molecule having a fourth nucleotide sequence being at least partially complementary to said first nucleotide sequence via a hybridization reaction between said at least first nucleotide sequence and said at least fourth nucleotide sequence, wherein said nucleic acid antisense strand is provided as follows:
- providing said nucleic acid target molecule,
- hybridizing at least one primer oligonucleotide to at least a first segment of said second nucleotide sequence, said at least first segment is located upstream and/or downstream of said first nucleotide sequence,
- extending said primer oligonucleotide by a nucleotide polymerase to generate said antisense nucleic acid strand, and that
said nucleic acid target molecule is a circular nucleic acid molecule.

The features, characteristics, advantages and embodiments specified for the method according to the invention apply likewise to the use according to the invention.

The invention is now further explained by means of embodiments resulting in additional features, characteristics and advantages of the invention. The embodiments are of pure illustrative nature and do not limit the scope or range of the invention. The features mentioned in the specific embodiments are features of the invention and may be seen as general features which are not applicable in the specific embodiment but also in an isolated manner in the context of any embodiment of the invention.

The invention is now further described and explained in further detail by referring to the following non-limiting examples and drawings:
- Fig. 1:: shows the reaction equilibrium of a circular nucleic acid molecule between the open, relaxed circular form and the intramolecular hairpin structure as well as the bimolecular hybridization structure;
- Fig. 2:: shows the reaction equilibrium of a circular nucleic acid molecule between the hairpin structure and the open, relaxed circular form without (A) and with (B) the hybridized antisense nucleic acid molecule;
- Fig. 3:: shows the hybridization of a circular nucleic acid molecule to a noncircular nucleic acid molecule hybridized on a solid surface, and the formation of a hairpin structure without (A) and with (B) the hybridized antisense nucleic acid molecule, and illustrates the involved nucleotide sequences;
- Fig. 4:: shows various options for the production of the antisense strand of the circular nucleic acid molecule via oligonucleotide synthesis (A), primer extension (B), and exonuclease activity (C);
- Fig. 5:: shows the result of an experimental validation of the method according to the invention.

### 1. Method according to the invention and background

Hybridization of nucleic acid molecules only occurs if a number of preconditions are fulfilled, such as (1) the hybridizing regions are sufficiently complementary to each other, (2) the length of the hybridizing regions is sufficiently large enough at the given reaction conditions, such as pH, ion strength, temperature etc., (3) the concentration of the nucleic acid molecules is sufficiently high enough at the given reaction conditions, (4) there is enough time available. There are also differences in the hybridization behavior if one hybridization partner is fixed to a solid surface; see Sekar et al. (2005), Comparative study of sequence-dependent hybridization kinetics in solution and on microspheres, Nucleic Acids Res. 14, 33(1):366-375, and Gao et al. (loc. cit.). Other preconditions are known to the skilled person.

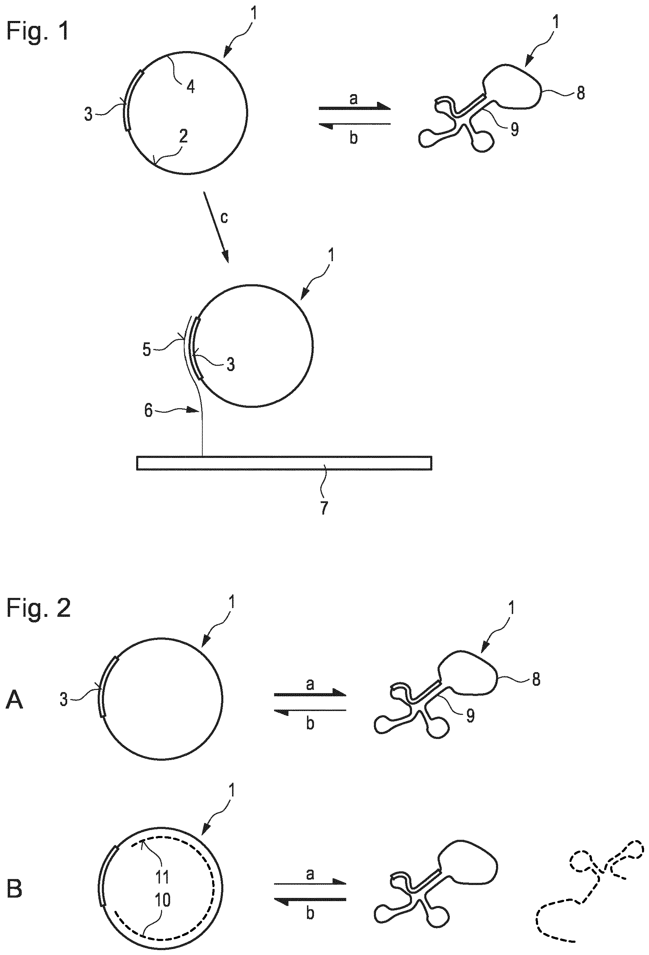

Hybridization is a bimolecular reaction of two complementary nucleic acid single strands. Nucleic acid single strands can, however, also hybridize to hairpin structures (Fig. 1). Hairpin structures are structure which can form within a nucleic acid single strand via hybridization of two complementary regions (Fig. 1, reaction a).

Hairpin structures are stabilized by supercoiling of DNA; see Brazda et al. (2011), Cruciform structures are a common DNA feature important for regulating biological processes, BMC Molecular Biology 12:33. Supercoiling of short DNA molecules can occur only in circular DNA so that hairpin structures are particularly favored in circular DNA.

For the hybridization of two regions to a hairpin structure again the above-mentioned hybridization conditions apply. However, the concentration of the nucleic acid single strand is of no or only minor importance since in a nucleic acid single strand both the target sequence and the complementary sequence are contained (monomolecular reaction).

The closer the two complementary regions are located on a single strand, the faster the hairpin formation occurs. The formation of the hairpin structure (Fig. 1, reaction a) is in equilibrium with the resolution of the hairpin structure (Fig. 1, reaction b). However, the formation of the hairpin structure is clearly favored as indicated by the bold arrow of the forward reaction a, Fig. 1. The formation of hairpin structures is for this reason also kinetically favored over a bimolecular hybridization of two complementary nucleic acid single strand molecules (kinetic balance model) (compare reaction a and reaction c in Fig. 1).

Hairpin structures consist of a hybridizing stem (Fig. 1, (9)) and a nonhybridizing loop (Fig. 1, (8)). If the stem structure in the hairpin is short and the hybridization of the two single-stranded molecules results in a long nucleic acid double-strand molecule as shown in Fig. 2B it results in a significantly higher energy gain (Gibbs energy or ΔG) because of the hybridization of the two long nucleic acid single strands than with the hybridization of the complementary regions on a single strand (thermodynamic balance model). In other words, in spite of the fast formation of the hairpin structure, in the long term the balance shifts to the long bimolecular nucleic acid double strands, i.e. reaction b is favored over reaction a in Fig. 2B.

To summarize, the capability of nucleic acid single strands to form hairpin structures has consequences for the formation of bimolecular nucleic acid double strands: (1.) the more and stabile hairpin structures can be formed, the slower nucleic acid single strands form bimolecular hybrids. (2.) The smaller the difference between ΔG resulting from the formation of the hairpin structure and the formation of the bimolecular nucleic acid double strand, the more inefficient and slower is the formation of the bimolecular double strand. This means, if the hybridizing region in the double strand has the similar length like in the hairpin structure only few bimolecular double strands are formed. (3.) With little differences between ΔG from the hairpin structure and the bimolecular nucleic acid double strand, there will always be both molecules in the reaction, the single strands with hairpin structure and the bimolecular nucleic acid double strands. According to the level of the ΔG difference, the percentage will be shifted to the single strands with hairpin structure or to the bimolecular nucleic acid double strands. This however means in any case that by the formation of the hairpin structure the effective concentration of the single strands available for the formation of the bimolecular double strand is reduced and the hybridization into a bimolecular hybrid is delayed. (4.) This particularly results in problems if one of the nucleic acid single strands is immobilized on a solid support, e.g. microarrays, NGS methods, and the other strand to be hybridized is in solution. Hybridizations to nucleic acids which are immobilized to a solid support occur significantly delayed. Gao et al. (loc. cit.) measured in different systems a 20 to 40 fold reduced hybridization speed if one of the nucleic acid strands to be hybridized was immobilized on a carrier. If e.g. short oligonucleotides are immobilized on a solid support and a long single strand being in solution should be hybridized thereto (i.e. array, NGS), the following situation comes up: The hybridizing region is short because of the shortness of the oligonucleotides which were immobilized on the carrier. Frequently, the hybridizing part of the immobilized oligonucleotides has a length of 15 to 35 nucleotides. On the other side, there are long nucleic acid single strands in solution. They have commonly a length of about 200 to 500 nucleotides. Long nucleic acid single strands have a higher tendency to form hairpin structures than short nucleic acid single strands. The nucleic acid single strands which form hairpin structures delay the hybridization speed and reduce the number of hybridizing double strands. When arrays are used on which long nucleic acid molecules are immobilized, e.g. BAC arrays, also the immobilized single strand is affected by the formation of hairpin structures.

In many application examples or methods significantly differently long nucleic acid single strands are used in a hybridization reaction. This has the consequence that the hybridizing region can only be as long as the respective shorter reaction partner. The respective longer strand can - in view of the capability to form hairpin structures - be described as follows: (1.) The longer a nucleic acid strand (excluded are mononucleotide single strands such as polyA, polyC, polyT, polyU, polyG, Polyl etc.), the higher the likelihood to form hairpin structures. (2.) The higher the percentage of bases which are complementary against each other (e.g. GC content > 50 % or AT content > 50 %), the higher the likelihood to form hairpin structures. (3.) The higher the GC content of a nucleic acid single strand, the higher the likelihood to form stable hairpin structures. For this reason, the formation of hairpin structures by the respective longer nucleic acid single strand can result in a significantly delayed or ineffective hybridization.

The present invention solves this problem by preventing or reducing the formation of hairpin structures on at least one of the nucleic acid single strand molecules involved in the hybridization.

The method according to the invention results in a higher hybridization efficiency. The higher hybridization efficiency allows the use of a fewer amount of the hybridizing nucleic acid molecules and a shortening of the hybridization time. The method according to the invention is of particular advantage for circular nucleic acid molecules which tend to form hairpin structures which are stabilized by supercoiling. In small nucleic acid molecules (size for example < 5000 nucleotides), a stable supercoiling basically only occurs in circular nucleic acid molecules. For this reason, the risk of stable hairpin structures is very high in circular nucleic acids where the method according to the invention is of particular advantage.

In Fig. 1, a circular first nucleic acid molecule (1) is shown consisting of a sense strand (2) that comprises a first nucleotide sequence (3) and a second nucleotide sequence (4). The first nucleotide sequence (3) can hybridize with a fourth nucleotide sequence (5) of a linear second nucleic acid molecule (6) immobilized on a solid support (7). The circular first nucleic acid molecule (1) forms hairpin structures with a loop (8) and a stem (9). The circular first nucleic acid molecule (1) is in equilibrium with the open, circular form (left) and the hairpin structure form (right). The equilibrium is shifted to the hairpin structure (right). The reaction a resulting in the hairpin structure is favored over the reaction b resulting in the open, circular form. As a result, the concentration of the open form of the circular first nucleic acid molecule (1) is reduced and the bimolecular hybridization to the linear second nucleic acid molecule (6) via the reaction c occurs in significantly reduced reaction speed.

As shown in Fig. 2A, the circular first nucleic acid molecule (1) with the first nucleotide sequence (3) which can hybridize to the linear second nucleic acid molecule (6) forms hairpin structures with a loop (8) and a stem (9). The equilibrium is then strongly shifted to the of the hairpin structure (right) if good hybridization conditions are given, e.g. sufficiently length of the hybridizing region, appropriate salt, pH and temperature conditions.

In Fig. 2B the circular first nucleic acid molecule (1) hybridizes with an antisense nucleic acid strand (10) having a third nucleotide sequence (11) so that no hairpin structure can form or the formation of the hairpin structure is significantly reduced. As the double strand formed after the hybridization of the antisense nucleic acid strand (10) extends over a hybridization region significantly longer than the hybridization region within a hairpin structure, the hairpin structure and reaction a is no longer the preferred structure. In contrast, reaction b is now favored.

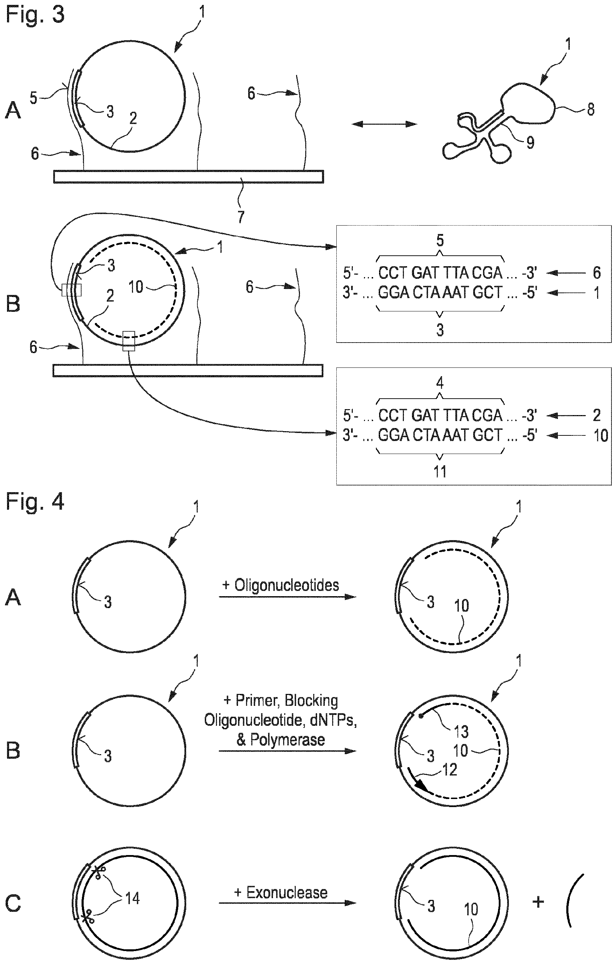

In Fig. 3A, the circular first nucleic acid molecule (1) is in equilibrium with a hairpin structure consisting of a loop (8) and a stem (9). The efficiency of the hybridization of the circular first nucleic acid molecule (1) via the first nucleotide sequence (3) to the linear second nucleic acid molecule (6) via the fourth nucleotide sequence (5) is reduced.

In Fig. 3B, the sense strand (2) of the circular first nucleic acid molecule (1) hybridizes to an antisense nucleic acid strand (10). The first nucleotide sequence (3) is still accessible since no hairpin structure is formed. As a result the circular first nucleic acid molecule (1) can hybridize to the linear second nucleic acid molecule (6) in a more efficient manner.

In Fig. 3B also the nucleotide sequences of the individual nucleic acid molecules and the respective strands are exemplarily illustrated. The second nucleotide sequence (4) of the sense strand (2) of the circular first nucleic acid molecule (1) is at least partially complementary to the third nucleotide sequence (11) of the antisense nucleic acid strand (10) of the circular first nucleic acid molecule (1). The fourth nucleotide sequence (5) of the linear second nucleic acid molecule (6) is at least partially complementary to the first nucleotide sequence (3) of the circular first nucleic acid molecule (1).

In Fig. 4A, the antisense nucleic acid (10) is synthesized via oligonucleotide synthesis and hybridizes to the circular first nucleic acid molecule (1). An oligonucleotide is synthesized in a manner that the first nucleotide sequence (3) serving for the hybridization to the linear second nucleic acid molecule (6) remains free.

In Fig. 4B two oligonucleotides (12, 13) are hybridized to the circular first nucleic acid molecule (1), framing the region of the subsequent antisense nucleic acid (11). The 5' or upstream located oligonucleotide (12) serves as primer for a primer extension. The 3' or downstream located oligonucleotide (13) is blocked at its 3' terminus and cannot be extended by a polymerase. Because of the use of a polymerase without strand displacement function and without 5'-3' exonuclease activity, the primer extension reaction blocks as soon as the polymerase during the synthesis has reached the 5' terminus of the 3' terminally-blocked oligonucleotide of the downstream located oligonucleotide (13).

In Fig. 4C, a circular nucleic acid double strand sample is provided. An exo-, endonuclease or glycosylase (14) is used to remove the part of the antisense strand that is hybridized to the first nucleotide sequence (3).

### 2. Experimental validation 1

It should be shown that by using a antisense nucleic acid a circular DNA (circle) can hybridize to oligonucleotides which were immobilized to a solid carrier material, in a more efficient manner. As a control, a hybridization without antisense nucleic acid is used. After hybridization, a rolling circle amplification (RCA) and a real-time PCR is performed to quantify the hybridization events.

Streptavidin plates (StreptaWell strips, Roche) coated with primer 1 for (biotin - aaaaaaaattcgacctatccttgcgcagctcgag; SEQ ID NO:1) and primer 1 rev (biotin - aaaaaaaaccatgaacaaaatgtgactcatatc; SEQ ID NO:2) were incubated with 0 or 50 pg of a circular DNA (sequence: atgacgatatgagtcacattttgttcatgggcatgacattgatacacagttagacga-taggacagtacattcgacctatccttgcgcagctcgagatgacg; SEQ ID NO:3) and 0 to 200 pg antisense nucleic acid (gtcatcgtcatctcgagctgcgcaaggataggtcgaatgtactgtcctatcgtctaactgtgtatcaatgtc; SEQ ID NO:4) in 50 µl of an appropriate buffer (50 mM Tris, pH 7,5; 10 mM MgCl₂, 100 mM NaCl, 0,0012% Tween 20) were incubated for 15 min at room temperature. The mixtures containing the circular DNA and the antisense nucleic acid were previously hybridized against each other. The following mixtures were used:

**Tab. 1: Reaction mixtures**

| Mixture No. | Amount of circular DNA | Amount of antisense nucleic acid |
|---|---|---|
| 1 and 2 | 50 pg | 0 pg |
| 3 and 4 | 50 pg | 20 pg |
| 5 and 6 | 50 pg | 60 pg |
| 7 and 8 | 50 pg | 200 pg |
| 9 and 10 | 0 pg | 0 pg |
| 11 and 12 | 0 pg | 20 pg |
| 13 and 14 | 0 pg | 60 pg |
| 15 and 16 | 0 pg | 200 pg |

After the hybridization of the samples with DNA that was immobilized on the surface, the supernatant was removed, washed and dissolved in 49 µl RCA reaction buffer (37 mM Tris pH 7,5; 50 mM KCI; 10 mM MgCl₂, 20 mM (NH₄)₂SO₄, 1 mM dNTP Mix, φ29 polymerase). The reaction was carried out for 2 hours at 38°C. Then the supernatant was removed and the surface was washed. The reaction was stopped after 1 h at 37°C with 50 µl of stop solution (von REPLI-g mini kit). The supernatant contains DNA previously immobilized on the surface. This DNA can now be detected by a real-time PCR (QuantiFast green kit) with appropriate primers [primer 2 forward: 5' ctg tgt atc aat gtc atg cc 3' (SEQ ID NO:5) and primer 2 reverse: 5' gtt aga cga tag gac agt aca 3' (SEQ ID NO:6)].

The result is shown in the following table 2.

**Tab. 2: CT values measured after real-time PCR**

| Mixture No. | CT | Stabw |
|---|---|---|
| 1 and 2 | 18.21 | 1.06 |
| 3 and 4 | 16.90 | 0.25 |
| 5 and 6 | 14.89 | 0.17 |
| 7 and 8 | 14.01 | 0.17 |
| 9 and 10 | 28.74 | 1.27 |
| 11 and 12 | 28.82 | 0.39 |
| 13 and 14 | 26.87 | 0.39 |
| 15 and 16 | 27.41 | 0.45 |

The table shows significantly reduced CT values for the mixtures in presence of circular DNA and antisense nucleic acid. When using 500 pg of antisense nucleic acid in the mixture, a CT value is obtained that is 4.2 cycles lower than in the mixture without antisense nucleic acid. This indicates an 18 fold increased hybridization efficiency. Without circular nucleic acid and with 500 pg antisense nucleic acid in the mixture, a CT value is obtained which is 13.4 cycles higher than in a mixture with present circular DNA. This indicates that the antisense nucleic acid itself does not contribute to the real-time PCR signal.

### 3. Experimental validation 2

It should be shown that by using a antisense nucleic acid a circular DNA (circle) can hybridize to oligonucleotides which were immobilized to a solid carrier material in a more efficient manner. As a control, a hybridization without antisense nucleic acid is used. After the hybridization, a rolling circle amplification (RCA) and then a staining of the amplified DNA with YOYO1 is carried out. A subsequent scanning of the amplified DNA results in a visual evaluation of the hybridization events.

Streptavidin-covered glass carriers coated with primer 1 for (biotin - SEQ ID NO:1), primer 1 ref (biotin - SEQ ID NO:2) and primer poly A (biotin - aaaaaaaa; SEQ ID NO:7) were incubated for 15 min at room temperature with 0 or 50 pg of a circular CNA (SEQ ID NO:3) and 0 to 200 pg antisense nucleic acid (SEQ ID NO:4) in 50 µl of an appropriate buffer (50 mM Tris, pH 7,5; 10 mM MgCl₂, 100 mM NaCl, 0,0012% Tween 20). The mixtures containing the circular DNA and the antisense nucleic acid were previously hybridized against each other. The following mixtures were used:

**Tab. 3: Mixtures used in the assay**

| Mixture No. | Amount of circular DNA | Amount of antisense nucleic acid |
|---|---|---|
| 1 | 50 pg | 0 pg |
| 2 | 50 pg | 60 pg |
| 3 | 50 pg | 200 pg |
| 4 | 0 pg | 200 pg |

After the hybridization of the samples to the DNA immobilized on the surface, the supernatant was removed, washed and dissolved in 49 µl of RCA reaction buffer (37 mM Tris pH 7,5; 50 mM KCI; 10 mM MgCI2, 20 mM (NH₄)₂SO₄, 1 mM dNTP Mix, φ29 polymerase). The reaction was carried out for 2 h at 38°C. Then the supernatant was removed and the surface was washed. Then the surface was covered with a 1:10,000 YOYO^{®}-1 iodide solution and incubated for 30 min. After washing the surface, the glass slide was scanned.

Then, the DNA was treated with 50 µl of DLB (from REPLI-g mini kit). The treatment was stopped after 1 h at 37°C with 50 µl of stop solution (from REPLI-g mini kit). The supernatant now contains DNA previously immobilized on the surface. This DNA can now be detected in a real-time PCR (QuantiFast SYBR green kit) with appropriate primers [primer 2 forward: (SEQ ID NO:5) und primer 2 reverse (SEQ ID NO:6)].

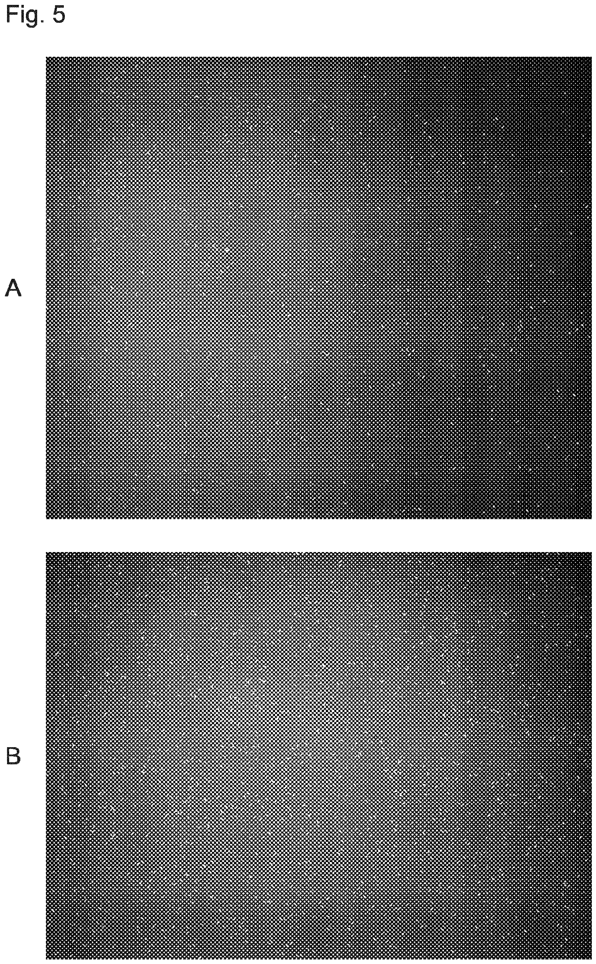

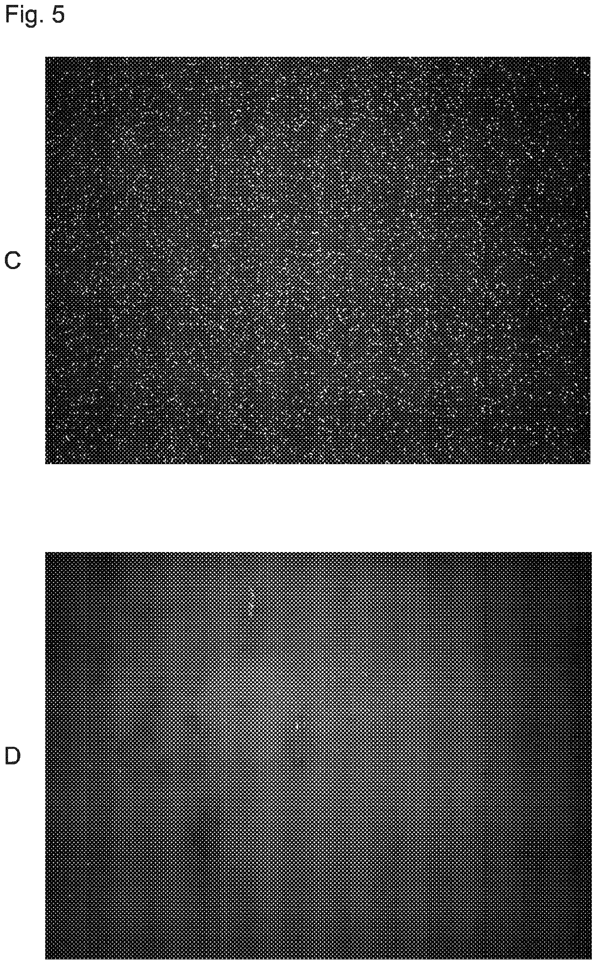

The result of the scanning is shown in Fig. 5 depicting sections of the scanned glass slides. The hybridization events are shown as small spots. It can be clearly seen that with increasing amounts of antisense nucleic acid, while remaining the amount of the circular DNA constant, the number of spots (hybridization events) increases. The sole presence of circular nucleic acid without presence of the antisense nucleic acid results in the lowest number of spots (hybridization events). Without the presence of the circular nucleic acid in the presence of antisense nucleic acid, no spots (hybridization events) can be seen.

**Tab. 4: Result from the real-time PCR**

| No. | CT | Stabw |
|---|---|---|
| 1 | 14.93 | 0.01 |
| 2 | 14.17 | 0.08 |
| 3 | 11.69 | 0.26 |
| 4 | 23.47 | 0.24 |

The table clearly shows lower CT values for the mixtures in presence of circular DNA and antisense nucleic acid. This indicates higher hybridization efficiency. Without circular nucleic acid and with 200 pg of antisense nucleic acid, a significantly higher CT value is obtained than in presence of circular DNA. This indicates that the antisense nucleic acid does not contribute to the real-time PCR signal.

### 4. Experimental validation 3

It should be shown that by using different lengths of antisense nucleic acids, a circular DNA (circle) can be hybridized to oligonucleotides, which were immobilized on a solid carrier material, in a more efficient manner. As a control hybridization with antisense nucleic acid was used. After the hybridization, rolling circle amplification (RCA) and a real-time PCR was carried out to quantify the hybridization events.

The already hybridized nucleic acid mixture consisting of 50 pg of circular DNA (circle) and 200 pg of antisense nucleic acid was hybridized to streptavidin plates (StreptaWell strips, Roche) which were coated with primer 1 for (biotin - SEQ ID NO:1) and primer 1 rev (biotin - SEQ ID NO:2) by using 50 µl of an appropriate buffer (50 mM Tris, pH 7,5; 10 mM MgCl₂, 100 mM NaCl, 0,0012% Tween 20).

Sequence of the circle: atgacgatatgagtcacattttgttcatgggcatgacattgata cacagttagacgataggacagtacattcgacctatccttgcgcagctcgagatgacg (SEQ ID NO:3)

Sequence of the antisense nucleic acids:
Nr. 1: gtcatcgtcatctcgagctgcgcaaggataggtcgaatgtactgtcctatcgtctaactgtgtatcaatgtc (SEQ ID NO:4)
Nr. 2: tcatctcgagctgcgcaaggataggtcgaatgtactgtcctatcgtctaactgtgta (SEQ ID NO:8)
Nr. 3: gagctgcgcaaggataggtcgaatgtactgtcctatcgtctaac (SEQ ID NO:9)
Nr. 4: gcaaggataggtcgaatgtactgtcctatc (SEQ ID NO:10)

The following mixtures were used

**Tab. 5: Mixtures used in the assay**

| Mixture | Circle | Antisense nucleotide |
|---|---|---|
| 1 | 50 pg | 0 pg |
| 2 | 50 pg | 200 pg No. 1 |
| 3 | 50 pg | 200 pg No. 2 |
| 4 | 50 pg | 200 pg No. 3 |
| 5 | 50 pg | 200 pg No. 4 |

After a hybridization period of 15 min, where the samples were hybridized to the DNA primers immobilized on the surface, the supernatant was removed and dissolved in 50 µl of RCA reaction buffer (37 mM Tris pH 7,5; 50 mM KCI; 10 mM MgCI2, 20 mM (NH)₂SO₄, 1 mM dNTP Mix, φ29 polymerase). The reaction was carried out for 2 h at 38°C. Then the supernatant was removed and the surface was washed. Then, the DNA was treated with 50 µl of DLB (from REPLI-g mini kit) on the surface. The treatment was stopped after 1 h at 37°C with 50 µl of stop solution (from REPLI-g mini kit). The supernatant now contains DNA that was previously immobilized on the surface. This DNA can now be detected in a real-time PCR (QuantiFast SYBR green kit) with appropriate primers [primer 2 forward (SEQ ID NO:5) und Primer 2 reverse (SEQ ID NO:6)].

The result is shown in the following table 6

**Tab. 6 CT values measured after real-time PCR**

| Mixture | Circle | Antisense nucleotide | CT | Variation coefficient |
|---|---|---|---|---|
| 1 | 50 pg | 0 pg | 20.85 | 0.0096 |
| 2 | 50 pg | 200 pg No. 1 | 17.47 | 0.0254 |
| 3 | 50 pg | 200 pg No. 2 | 16.61 | 0.0239 |
| 4 | 50 pg | 200 pg No. 3 | 17.02 | 0.0239 |
| 5 | 50 pg | 200 pg No. 4 | 17.78 | 0.0187 |

The table clearly shows lower CT values for the mixtures with presence of circular DNA and antisense nucleic acids of different lengths. The antisense nucleic acids with different lengths cover the circular nucleic acid to different extents. The percentage indicates the extent of coverage of the circular nucleic acid by the antisense nucleic acid. The percentage in parentheses indicates the coverage of the circular nucleic acid by the antisense nucleic acid and the immobilized primer 1 rev which must hybridize to the circle.

**Tab. 7: Coverage of the circle by antisense nucleic acid**

| Antisense nucleic acid | Length (bp) antisense nucleic acid | Length (bp) circle | Length (bp) of the hybridized region between circle and immobilized primer 1 | Coverage circle by the antisense nucleic acid |
|---|---|---|---|---|
| No. 1 | 72 | 101 | 25 | 71% (96%) |
| No. 2 | 58 | 101 | 25 | 57% (82%) |
| No. 3 | 44 | 101 | 25 | 44% (68%) |
| No. 4 | 30 | 101 | 25 | 30% (54%) |

If 400 pg of antisense nucleic acid is used in the mixture, a CT value is obtained which is up to 4.2 cycles lower than in a mixture without antisense nucleic acid. This indicates an 18 fold higher hybridization efficiency. Even with strongly shortened antisense nucleic acid, which only results in coverage of 30 % of the circular nucleic acid, still a CT yield of > 3 cycles is reached.

### Sequences

SEQ ID NO: 1
   aaaaaaaatt cgacctatcc ttgcgcagct cgag
SEQ ID NO: 2
   aaaaaaaacc atgaacaaaa tgtgactcat atc
SEQ ID NO: 3 
atgacgatat gagtcacatt ttgttcatgg gcatgacatt gatacacagt tagacgatag gacag-
tacat tcgacctatc cttgcgcagc tcgagatgac g
SEQ ID NO: 4 
gtcatcgtca tctcgagctg cgcaaggata ggtcgaatgt actgtcctat cgtctaactg
tgtatcaatg tc
SEQ ID NO: 5
   ctgtgtatca atgtcatgcc
SEQ ID NO: 6
   gttagacgat aggacagtac a
SEQ ID NO: 7
   aaaaaaaa
SEQ ID NO: 8
   tcatctcgag ctgcgcaagg ataggtcgaa tgtactgtcc tatcgtctaa ctgtgta
SEQ ID NO: 9
   gagctgcgca aggataggtc gaatgtactg tcctatcgtc taac
SEQ ID NO: 10
   gcaaggatag gtcgaatgta ctgtcctatc

## Claims

1. A method for hybridizing a nucleic acid molecule, comprising
i) providing a reaction mixture comprising
- at least one first nucleic acid molecule comprising at least partially hybridized against each other
- a sense nucleic acid strand having
- at least a first nucleotide sequence, and
- at least a second nucleotide sequence;
- an antisense nucleic acid strand having at least a third nucleotide sequence being at least partially complementary to said a least second nucleotide sequence and being at least partially non-complementary to said at least first nucleotide sequence;
- at least one second nucleic acid molecule having a fourth nucleotide sequence being at least partially complementary to said first nucleotide sequence;
- hybridization buffer;
ii) incubating said reaction mixture under conditions allowing said at least one first nucleic acid molecule to hybridize to said at least one second nucleic acid molecule via a hybridization reaction between said at least first nucleotide sequence and said at least fourth nucleotide sequence,
**characterized in that** said at least one first nucleic acid molecule is provided as follows:
- providing said sense nucleic acid strand,
- hybridizing at least one primer oligonucleotide to at least a first segment of said second nucleotide sequence, said at least first segment is located upstream and/or downstream of said first nucleotide sequence,
- extending said primer oligonucleotide by a nucleotide polymerase to generate said antisense nucleic acid strand, and
said least one second nucleic acid molecule is linked to a solid support.

2. Method of claim 1, **characterized in that** said at least one first nucleic acid molecule is a circular nucleic acid molecule.

3. Method of claim 1 or 2, **characterized in that** before and/or during said extension a blocking oligonucleotide is hybridized to at least a second segment of said second nucleotide sequence, said second segment is located downstream of said first nucleotide sequence, preferably said polymerase exhibits no strand displacement activity and no 5'-3' exonuclease activity.

4. Method of any of the preceding claims, **characterized in that** said fourth nucleotide sequence is longer than said first nucleotide sequence.

5. Method of any of the preceding claims, **characterized in that** said at least one first nucleic acid molecule and said at least one second nucleic acid molecule are DNA.

6. Method of any of the preceding claims, **characterized in that** said at least one second nucleic acid molecule is a single stranded nucleic acid molecule.

7. Method of any of the preceding claims, comprising the following further step:
iii) removing said antisense strand,

8. Method of claim 7, **characterized in that** the removal of said antisense strand is realized by denaturation and/or via a strand displacement by using a polymerase with strand displacement activity.

9. Method of any of the proceedings claims, **characterized in that** said solid support comprises a material selected from the group consisting of: metal, glass, silica, plastics, and/or preferably is selected from the group consisting of: chips, beads, capillaries.

10. Use of a nucleic acid antisense strand having a third nucleotide sequence being at least partially complementary to a second nucleotide sequence and being at least partially non-complementary to a first nucleotide sequence, to reduce and/or avoid the formation of hairpin structures of a nucleic acid target molecule comprising said first nucleotide sequence and said second nucleotide sequence, said nucleic acid target molecule intended to be hybridized to a nucleic acid probe molecule having a fourth nucleotide sequence being at least partially complementary to said first nucleotide sequence via a hybridization reaction between said at least first nucleotide sequence and said at least fourth nucleotide sequence,
**characterized in that** said nucleic acid antisense strand is provided as follows:
- providing said nucleic acid target molecule,
- hybridizing at least one primer oligonucleotide to at least a first segment of said second nucleotide sequence, said at least first segment is located upstream and/or downstream of said first nucleotide sequence,
- extending said primer oligonucleotide by a nucleotide polymerase to generate said antisense nucleic acid strand, and that
said nucleic acid target molecule is a circular nucleic acid molecule.

## Patentansprüche

1. Verfahren zur Hybridisierung eines Nukleinsäuremoleküls, das Folgendes aufweist:
i) Bereitstellen eines Reaktionsgemisches, das Folgendes aufweist:
- zumindest ein erstes Nukleinsäuremolekül, das Folgendes, zumindest teilweise gegeneinander hybridisiert, aufweist:
- einen Sinn-Nukleinsäurestrang mit
- zumindest einer ersten Nukleotidsequenz, und
- zumindest einer zweiten Nukleotidsequenz;
- einen Gegensinn-Nukleinsäurestrang mit zumindest einer dritten Nukleotidsequenz, die zumindest teilweise komplementär zu der zumindest zweiten Nukleotidsequenz ist und zumindest teilweise nicht komplementär zu der zumindest ersten Nukleotidsequenz ist;
- zumindest ein zweites Nukleinsäuremolekül mit einer vierten Nukleotidsequenz, die zumindest teilweise komplementär zu der ersten Nukleotidsequenz ist;
- Hybridisierungspuffer;
ii) Inkubieren des Reaktionsgemischs unter Bedingungen, die es dem zumindest einen ersten Nukleinsäuremolekül erlauben, an das zumindest eine zweite Nukleinsäuremolekül über eine Hybridisierungsreaktion zwischen der zumindest ersten Nukleotidsequenz und der zumindest vierten Nukleotidsequenz zu hybridisieren,
**dadurch gekennzeichnet, dass** das zumindest eine erste Nukleinsäuremolekül wie folgt bereitgestellt wird:
- Bereitstellen des Sinn-Nukleinsäurestrangs,
- Hybridisieren von zumindest einem Primer-Oligonukleotid mit zumindest einem ersten Segment der zweiten Nukleotidsequenz, wobei das zumindestens erste Segment stromaufwärts und/oder stromabwärts der ersten Nukleotidsequenz lokalisiert ist,
- Verlängern des Primer-Oligonukleotids durch eine Nukleotidpolymerase, um den Gegensinn-Nukleinsäurestrang zu erzeugen, und
wobei das zumindest eine zweite Nukleinsäuremolekül an einen festen Träger gebunden ist.

2. Verfahren nach Anspruch 1, **dadurch gekennzeichnet, dass** das zumindest eine erste Nukleinsäuremolekül ein zirkuläres Nukleinsäuremolekül ist.

3. Verfahren nach Anspruch 1 oder 2, **dadurch gekennzeichnet, dass** vor und/oder während der Verlängerung ein blockierendes Oligonukleotid an zumindest ein zweites Segment der zweiten Nukleotidsequenz hybridisiert wird, wobei das zweite Segment stromabwärts der ersten Nukleotidsequenz lokalisiert ist, wobei die Polymerase vorzugsweise keine Strangverdrängungsaktivität und keine 5'-3'-Exonukleaseaktivität aufweist.

4. Verfahren nach einem der vorhergehenden Ansprüche, **dadurch gekennzeichnet, dass** die vierte Nukleotidsequenz länger ist als die erste Nukleotidsequenz.

5. Verfahren nach einem der vorhergehenden Ansprüche, **dadurch gekennzeichnet, dass** das zumindest eine erste Nukleinsäuremolekül und das zumindest eine zweite Nukleinsäuremolekül DNA sind.

6. Verfahren nach einem der vorhergehenden Ansprüche, **dadurch gekennzeichnet, dass** das zumindest eine zweite Nukleinsäuremolekül ein einzelsträngiges Nukleinsäuremolekül ist.

7. Verfahren nach einem der vorhergehenden Ansprüche, das den folgenden weiteren Schritt aufweist:
iii) Entfernen des Gegensinn-Strangs.

8. Verfahren nach Anspruch 7, **dadurch gekennzeichnet, dass** die Entfernung des Gegensinn-Strangs durch Denaturierung und/oder über eine Strangverdrängung unter Verwendung einer Polymerase mit Strangverdrängungsaktivität erfolgt.

9. Verfahren nach einem der vorhergehenden Ansprüche, **dadurch gekennzeichnet, dass** der feste Träger ein Material aufweist, das ausgewählt ist aus der Gruppe bestehend aus: Metall, Glas, Siliziumdioxid, Kunststoff, und/oder vorzugsweise ausgewählt ist aus der Gruppe bestehend aus: Chips, Beads, Kapillaren.

10. Verwendung eines Nukleinsäure-Gegensinn-Strangs mit einer dritten Nukleotidsequenz, die zumindest teilweise komplementär ist zu einer zweiten Nukleotidsequenz und zumindest teilweise nicht komplementär ist zu einer ersten Nukleotidsequenz, zur Verringerung und/oder Vermeidung der Bildung von Haarnadelstrukturen eines Nukleinsäure-Zielmoleküls, das die erste Nukleotidsequenz und die zweite Nukleotidsequenz aufweist, wobei das Nukleinsäure-Zielmolekül dazu bestimmt ist, an ein Nukleinsäure-Sondenmolekül mit einer vierten Nukleotidsequenz, die zumindest teilweise komplementär zu der ersten Nukleotidsequenz ist, über eine Hybridisierungsreaktion zwischen der zumindest ersten Nukleotidsequenz und der zumindest vierten Nukleotidsequenz hybridisiert zu werden, **dadurch gekennzeichnet, dass** der Nukleinsäure-Gegensinn-Strang wie folgt bereitgestellt wird:
- Bereitstellen des Nukleinsäure-Zielmoleküls,
- Hybridisieren von zumindest einem Primer-Oligonukleotid an zumindest ein erstes Segment der zweiten Nukleotidsequenz, wobei das zumindest erste Segment stromaufwärts und/oder stromabwärts der ersten Nukleotidsequenz lokalisiert ist,
- Verlängern des Primer-Oligonukleotids durch eine Nukleotidpolymerase, um den Gegensinn-Nukleinsäurestrang zu erzeugen, und dass
das Nukleinsäure-Zielmolekül ein zirkuläres Nukleinsäuremolekül ist.

## Revendications

1. Procédé d'hybridation d'une molécule d'acide nucléique, comprenant
i) la fourniture d'un mélange réactionnel comprenant
- au moins une première molécule d'acide nucléique comprenant, au moins partiellement hybridés les uns contre les autres,
- un brin d'acide nucléique sens ayant
- au moins une première séquence nucléotidique, et
- au moins une deuxième séquence nucléotidique ;
- un brin d'acide nucléique antisens ayant au moins une troisième séquence nucléotidique qui est au moins partiellement complémentaire de ladite au moins une deuxième séquence nucléotidique et qui est au moins partiellement non complémentaire de ladite au moins une première séquence nucléotidique ;
- au moins une deuxième molécule d'acide nucléique ayant une quatrième séquence nucléotidique qui est au moins partiellement complémentaire de ladite première séquence nucléotidique ;
- un tampon d'hybridation ;
ii) l'incubation dudit mélange réactionnel dans des conditions permettant à ladite au moins une première molécule d'acide nucléique de s'hybrider à ladite au moins une deuxième molécule d'acide nucléique par une réaction d'hybridation entre ladite au moins une première séquence nucléotidique et ladite au moins une quatrième séquence nucléotidique,
**caractérisé en ce que** ladite au moins une première molécule d'acide nucléique est fournie comme suit :
- la fourniture dudit brin d'acide nucléique sens,
- l'hybridation d'au moins un oligonucléotide amorce à au moins un premier segment de ladite deuxième séquence nucléotidique, ledit au moins un premier segment est situé en amont et/ou en aval de ladite première séquence nucléotidique,
- l'extension dudit oligonucléotide amorce par une nucléotide polymérase pour générer ledit brin d'acide nucléique antisens, et
ladite au moins une deuxième molécule d'acide nucléique est liée à un support solide.

2. Procédé de la revendication 1, **caractérisé en ce que** ladite au moins une première molécule d'acide nucléique est une molécule d'acide nucléique circulaire.

3. Procédé de la revendication 1 ou 2, **caractérisé en ce qu'**avant et/ou pendant ladite extension, un oligonucléotide de blocage est hybridé à au moins un deuxième segment de ladite deuxième séquence nucléotidique, ledit deuxième segment est situé en aval de ladite première séquence nucléotidique, de préférence ladite polymérase ne présente aucune activité de déplacement de brin et aucune activité exonucléase 5'-3'.

4. Procédé de l'une des revendications précédentes, **caractérisé en ce que** ladite quatrième séquence nucléotidique est plus longue que ladite première séquence nucléotidique.

5. Procédé de l'une des revendications précédentes, **caractérisé en ce que** ladite au moins une première molécule d'acide nucléique et ladite au moins une deuxième molécule d'acide nucléique sont de l'ADN.

6. Procédé de l'une des revendications précédentes, **caractérisé en ce que** ladite au moins une deuxième molécule d'acide nucléique est une molécule d'acide nucléique simple brin.

7. Procédé de l'une des revendications précédentes, comprenant l'étape supplémentaire suivante :
iii) l'élimination dudit brin antisens.

8. Procédé de la revendication 7, **caractérisé en ce que** l'élimination dudit brin antisens est réalisée par dénaturation et/ou par un déplacement de brin en utilisant une polymérase à activité de déplacement de brin.

9. Procédé de l'une des revendications précédentes, **caractérisé en ce que** ledit support solide comprend un matériau choisi dans le groupe constitué par : un métal, du verre, la silice, des matières plastiques, et/ou de préférence est choisi dans le groupe constitué par : des puces, des billes, des capillaires.

10. Utilisation d'un brin antisens d'acide nucléique ayant une troisième séquence nucléotidique qui est au moins partiellement complémentaire d'une deuxième séquence nucléotidique et qui est au moins partiellement non complémentaire d'une première séquence nucléotidique, pour réduire et/ou éviter la formation de structures en épingle à cheveux d'une molécule cible d'acide nucléique comprenant ladite première séquence nucléotidique et ladite deuxième séquence nucléotidique, ladite molécule cible d'acide nucléique étant destinée à être hybridée à une molécule sonde d'acide nucléique ayant une quatrième séquence nucléotidique qui est au moins partiellement complémentaire de ladite première séquence nucléotidique par une réaction d'hybridation entre ladite au moins une première séquence nucléotidique et ladite au moins une quatrième séquence nucléotidique,
**caractérisée en ce que** ledit brin antisens d'acide nucléique est fourni comme suit :
- la fourniture de ladite molécule cible d'acide nucléique,
- l'hybridation d'au moins un oligonucléotide amorce à au moins un premier segment de ladite deuxième séquence nucléotidique, ledit au moins un premier segment est situé en amont et/ou en aval de ladite première séquence nucléotidique,
- l'extension dudit oligonucléotide amorce par une nucléotide polymérase pour générer ledit brin d'acide nucléique antisens, et **en ce que**
ladite molécule cible d'acide nucléique est une molécule d'acide nucléique circulaire.
